# EUROPEAN PATENT SPECIFICATION

(11) **EP 0 729 731 B1**
(45) Date of publication and mention of the grant of the patent: **02.06.1999**
(21) Application number: 95500027.8
(22) Date of filing: 03.03.1995
(51) Int. Cl.: A61B 17/58

(54) **Spinal column fixation system**
System zum Fixieren einer Wirbelsäule
Système de fixation de colonne vertébrale

(43) Date of publication of application: 04.09.1996
(73) Proprietor: INDUSTRIAS QUIRURGICAS DE LEVANTE, S.A., 46988 Paterna - Valencia (ES)
(72) Inventor: Cogollos, Bernardo, E-46988 Paterna, Valencia (ES); Monfort Vicente, Victor, E-46988 Paterna, Valencia (ES)
(74) Representative: Urizar Anasagasti, José Antonio

(56) References cited:
- EP-A- 0 468 264
- EP-A- 0 553 424
- EP-A- 0 593 321
- US-A- 5 122 131
- US-A- 5 254 118

## Description

The present invention refers to a new fixation system for surgical implants of vertebral column based on attachment by means of pedicular screw.

Comparing what there is manufactured until the present times one comes to various conclusions and advantages such as:
- Physical and mechanical characteristics of the material used.

The physical and mechanical characteristics in a column implant fixation system have a great importance. As an example, the alloy of Ti-6Al-4V has higher hardness than the stainless steel AISI 316 LVM.

For equal values of fracture tension and higher values of elastic limit in the Ti alloy, this means that based on equal sections, the elastic performance of the Ti alloy is produced in a larger field of tensions.

The difference between the elasticity modules of the bone and that of the AISI 316 LVM means that for a given effort, the bone will suffer different deformations than the fixation system, with subsequent dangers. The difference between the bone and the Ti-6Al-4V is approximately the half, thus considerably reducing said dangers.

The resistance to stress is an important characteristic of the prime material which is used in these cases, particularly if it is going to be submitted to cyclic loads.

The density is a physical characteristic which should be borne in mind as a low density value as that of the Ti (almost half of that of the AISI 316 LVM) provides remarkably slighter elements to the benefit of implants carrying patients.

In the case of surgical implants, any element introduced in the human body must have a good resistance against corrosion that might be originated within the organism's fluids. Through experiences and studies carried out, the remarkable behaviour of the Ti on corrosive ambients is known.

Besides the material, there were a series of inconveniences such as having a threaded system for the assembling of the modular fixation system and that a mounting was also necessary prior to the fitting.

The system of threaded rod, previously used, is not precisely the most confortable for using once the rods are placed in situ. It was really painful and slow the screwing of the nuts and their handling with the wrenches usually available within the instrumentals, including the ratchet wrench which has a difficult application when the rod is placed under a dihedral angle.

It was thus a question of patience and of undertaking a previous mounting as approximate as possible to the definite disposition of the system.

The mounting before the fitting was really an inconvinient as sometimes it was necessary to change the type of the prior mounting several times until the desired modular disposition is obtained.

Also previously there existed two types of pedicular shanks, by traction, of obliged use in those cases where to the transpedicular fixation, one has to associate any anteroposterior reduction operation and pedicular stems for fractures, of compulsory use in treatments by posterior access of vertebral fractures, using one or another stem according to the circumstances.

Solutions, more or less successful in obtaining a good osteosynthesis, have been provided over the years by an infinit number of methods. Said methods overcame the problem exclusively by posterior access in some cases, or by front access in others.

Consequently, a method was needed capable of establishing a solid vertebral mounting by posterior access which would achieve, among fractures and spondylolisthesis, to reduce, fix and stabilize the time necessary for the simple consolidation of a simple form.

In earlier times, in order to perform a reduction, a support point was necessary in order to be able to create a first grade lever bar. For fixing it was necessary to have a solid vertebral anchoring, the pedicule being the best support point. For stabilizing it was needed to create a mechanical mounting capable of maintaining itself stable during the time necessary for the mending of the injury.

Patent EP-A-O 593 321 "Systeme d'osteosynthese rachidiene" refers to a fixation system comprising a pedicular stem having a tapered threaded tip and the resto of its length being of constant diameter cylinder shape with plain exterior surface; this stem is fastened to a setscrew that exerts a pressure on a round bar which stays loose within a large cube. The implant of this system turns out to be complicate and does not provide a good lasting connection with the vertebral column.

In order to resolve the aforementioned problems, the use of a fixation system of column, object of the present invention, is proposed , by posterior access, reducing the spondylolisthesis by direct traction on the pedicule, by means of the pedicular stems. It reduces the fractures by means of the powerful lever bar that the pedicular stems constitute together with the bars.

It fixes, by means of the screws which are embedded in the whole vertebral pedicule, inmobilizing completely the vertebra, both in the flexo-extention and the rotation efforts. It stabilizes, due to the solid mounting established by the screw heads, the fittings and by the bars capable of being assembled by means of tightening and blocking screws.

The most appropriate material, as it has already been seen, due to its mechanical and physical properties and to its resistance against corrosion is that of the alloy Ti-6Al-4V which is preferably used for the object of the present invention, pertaining in turn to the group of materials called biomaterials, that is, to those which are compatible before the human organism as well as the organism before them. On the other hand, this alloy does not disturb the NMR (Nuclear Magnetic Resistance) images.

With the fixation system, object of the invention, a very appropriate, simple and rapid solution is achieved during the implantation.

The operating time is very much simplified and the operations for fitting the fixer to the corresponding vertebras are extraordinarily facilitated.

The utilization range is extended as with this system is possible to conform the curvature of the rod to any eventual curve of vertebral column, not being this curve an obstacle for the correct fastening and blocking of the fixer in any position.

On the other hand, with this fixation system the traction and fracture stems are unified, that is, with only one pedicular stem model the needs for the cases where one stem or the other is required is covered, since the components necessary for fitting operation of the fixer are simplified. In this unique pedicular stem the pulling out (fraction) resistance and the flexion resistance (fracture) are combined.

In addition to accomplishing the fundamental objectives of a medular fixation device which are those of fixing, stabilizing and reducing, this system offers a series of qualities such as the versatility; that is, it enables the possibility of being adapted either to vertebral bodies or to posterior arcs (pedicules). It is also modular, it can be divided in different elements or modules which provide for multiple combinations with very few elements. It is combinable with other systems of vertebral instrumentation. It is of a great handling and learning ease for the surgeons and instrumentists and it avoids, where possible, rigid external contentions as plaster beddings, plastered corsets, etc.

As in all instrumental surgery of vertebral column, the infective pathology remains out of the indication possibilities of this instrumentation. The pathology where this modular vertebral fixer can be used is in the case of vertebral fractures, primitive and metatarsical tumorations, deformation sequelas from the traumatisms, congenital deformations (kyphoscoliosis), lesions and degenerative disc diseases, postchirurgical inestabilities, kyphosis ans scoliosis of all the aetiologies, spondylolysis and spondylolisthesis, surgery of the vertebral reumatism, support on arthrodesis of any kind.

The invention will be better understood with the help of the following description, only given as a non-limitative example type, being related to the enclosed drawings, wherein:
Figure 1 shows a fittin assembly with the bolt-screw, bar, nut and pedicular stem.
Figure 2 shows different views of a fitting.
Figure 3 shows a bolt-screw with cylindrical recess.
Figure 4 shows a cap nut.
Figure 5 shows a bar.

The new fixation system bar (1)-fitting (2) consists of an attachment by means of a tightening screw. Two new elements then appear: bolt-screw with cylindrical recess (3) perpendicularly situated onto the bar and nut (4) which is threaded at the end of the bolt-screw (3).

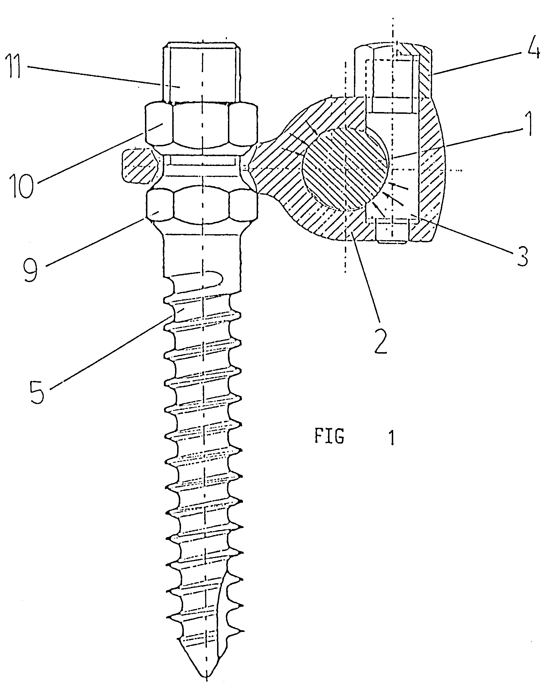

Once the bar (1) is introduced in the fitting (2) with bolt-screw (3), the bar (1) can be blocked due to she traction obtained by rotating the nut (4), thus achieving a superficial friction of the cylindrical recess of the bolt-screw (3) with the bar surface, as it can be seen by arrows indicating the friction zone in figure 1.

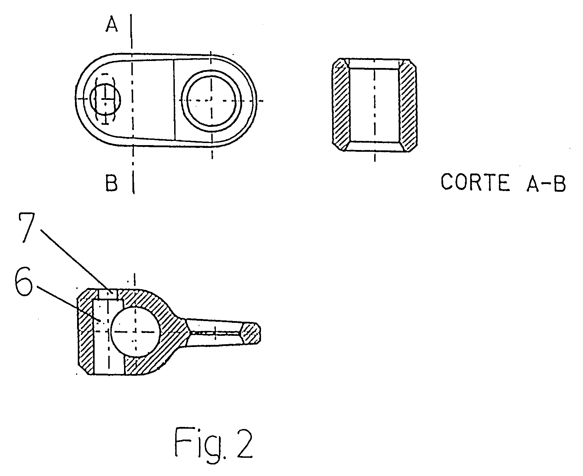

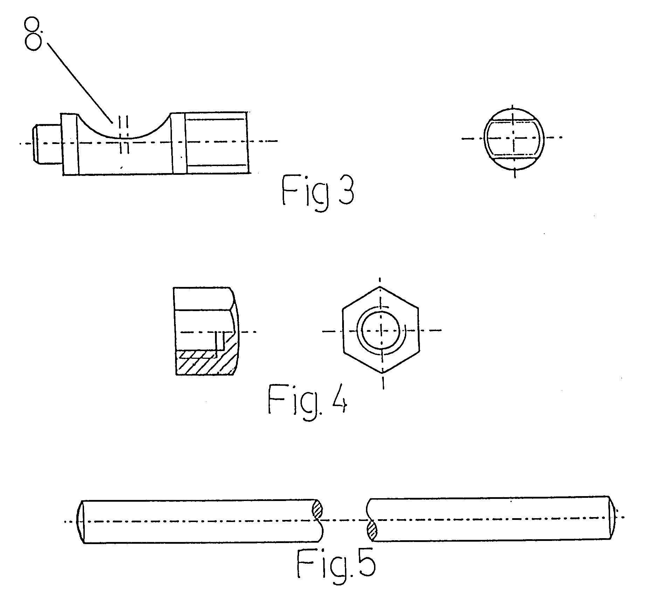

Figure 2 represents a simple fitting which incorporates a transversal cylindrical hollow space (6) with the purpose of allocating the bolt-screw (3) and another shorter guiding means (7) which is used as a guide for the bolt-screw such that it acquires a unique position at its location, as distinguishing parts with respect to a normal fitting, as this fitting can be any of the ones included in modular systems. In figure 3, the bolt-screw can be seen which carries a cylindrical recess (8) through the hollow space of which passes the blank bar (1) and within the fitting a pressure is exerted between the recess (8) and the bar (1), thus producing the fixation of the system. An end of the bolt-screw (3) is threaded in order to fit with the nut (4), which upon tightening at this end of the screw exerts a traction on the latter pulling it up and provoking pressure of the recess (8) against the bar (1). At the other end it carries a salient (male) which matches to the form of the guiding means (7) of the fitting (2), the function of which is to guide the bolt-screw (3) with a single position.

In figure 5, the coupling bar (1) is shown which can present different lengths. This bar has preferably been formed with plain surface, that is, it is not threaded, thus avoiding the tedious task of screwing nuts on the same before and during the assembling operation.

In figure 1 the pedicular stem (5) is shown which as already commentated is unique, that is, it is utilized either for the cases where it is necessary to use traction stem or for those in which fracture stems are placed.

This new pedicular stem will preferably have a variable threading pitch with a higher tooth height at the end point in order to increase the resistance against pulling-out and a larger core at the end to increase the resistance against flexion. The diameter at an intermediate zone is to increase its resistance.

This stem incorporates an integrated nut (9) which will act as counternut to the nut (10) which is screwed onto the stem threaded end (11) of the stem, after placing the fitting (2) on the stem (5).

Another advantage of this stem is that it does not need to be shortened.

The utilized material for all these components is preferably of Ti-6Al-4V.

Resuming the found advantages provided by this device, on the one hand there exists the facility in handling the elements during implant as well as the facility of learning by the surgeons and instrumentists. The number of the implanted components in the device is reduced, thus letting in turn the possibility of combination with other systems. The screws utilized for tightening the device are standard. The instrumental is simple and basic in the inventory of an operation room. It allows the simultaneous correction and stabilization, the simultaneous reduction and neutralization, at the same time that it provides solid and powerful stabilizations. It is versatile and permits multiple combinations, in addition to making premounting, unnecessary.

A practical embodiment of this device is the medular canal stenosis.

Once the zone for inserting the implant is located, the pedicular stems (5) are screwed on the pedicules, the bar (1) is bended such that it obtains the form of the final position in the implant; once bent, the fittings (2) are passed through the bar (1). This assembly of bar and fittings is fitted to the pedicular screws (5), then the stem nuts (10) are tightened. Once mounted the compression or distraction of the system is carried out in cases where it is necessary and finally the fitting pins are fixed.

Once sufficiently described the nature of the present invention, as well as the mode to carry it out to the practice, it only remains to be added that in the whole and parts of which it is composed of, it is possible to introduce changes of form, materials and disposition, provided that said alterations do not vary substantially the characteristics of the invention which are claimed herewith as follows.

## Claims

1. Fixation system of column constituted by a fitting of vertebral column (2), a pedicular stem (5) which can be used in the case of a traction as well as in the case of a fracture, a bolt-screw (3), a nut (4) and a cylinder shaped bar (1) with flat exterior surface, characterised in that the bolt-screw is provided with a cylindrical recess (8).

2. Fixation system of column, according to the first claim, characterised in that the pedicular stem is provided with variable thread, with higher depth on tip and lower depth on thread end due to core conicity and constant exterior diameter.

3. Fixation system of column, according to the first claim, characterised in that the bolt screw (3) is provided with a recess (8) matching the plain bar (1) and being, in conjunction with the bar, the element which exerts a pressure on the blocked device.

4. Fixation system of column, according to the first claim, characterised in that the bolt-screw (3) is provided with guiding means (7) allowing an unique position once said bolt-screw has been positioned in the fitting (2).

5. Fixation system of column, according to the first claim, characterised in that the bolt-screw (3) is preformed so that when acting on the screw, said screw is displaced and a pressure exerted on the bar (1) achieving the blocking of the system, the bolt screw (3) having a threaded end with the nut (4) connected to it, and abutting on fitting (2) pulls the bolt-screw (3) upwards, exerting the required pressure between the bar (1) and the cylindrical recess (8).

## Patentansprüche

1. Wirbel säulenbefestigungssystem, bestehend aus einer Verkoppelung der wirbelsäule (2), einem Schaft (5), den man sowohl im Bruchfall als auch im Zugspannungsfall benutzen kann, einer Durchsteckschraube (3), einer Mutter (4), und einer Stange (1) mit glatter Aussenöberfläche, dadurch gekennzeichnet, dass es mit einem zylindrischen Kavett (8) versehen ist.

2. Wirbel säulenbefestigungssystem nach dem erstem Patentanspruch, dadurch gekennzeichnet, dass der Schaft (5) ein veränderliches Gewinde mit höherer Verzahnung an der Spitze, und Kleinerer am Gewindeende besitzt, verursacht dadurch dass der Innenkern kegelförmig ist und das Äussere einen konstanten Durchmesser hat.

3. Wirbel säulenbefestigungssystem nach dem ersten und dem vohergehenden Patentanspruch, dadurch gekennzeichnet, dass die Durchsteckschraube (3) einen Kavett (8) einbegreift welcher im Zusammenhang mit der Form der glatten Stange (1) steht, und zusammen mit der Stange (1) das Druckelement ist, welches nach der Blockierung die Kraft über die Einrichtung ausübt

4. Wirbel säulenbefestigungssystem nach dem erstem Patentanspruch, dadurch gekennzeichnet dass die Durchsteckschraube (3) eine Führung (7) besitzt, welche die einzige Stellung nach Anlegung der Verkoppelung (2) erlaubt.

5. Wirbel säulenbefestigungssystem nach dem erstem Patentanspruch, dadurch gekennzeichnet dass die Durchsteckschraube (3) so vorgeformt ist, dass bei der Einwirkung auf die Schraube eine Verschiebung derselben verursacht wird, die eine Kraft auf die Stange (1) ausübt, und somit eine Blockierung des Systems erreicht wird, die Durchsteckschraube (3) besteht aus einem Gewindeende auf dem die Mutter (4) wirkt, die an der Verkoppelung (2)anstösst, damit an der Durchsteckschraube (3) nach oben zieht, und somit den gewünschten Druck zwischen der Stange (1) und dem Kavett (8) erreicht.

## Revendications

1. Système de fixation de colonne constitué par un accouplement de colonne vertébrale (2), une queue pédiculaire (5) qui peut être utilisée tant pour des cas de traction que pour des cas de fracture, un vis à pas (3), un écrou (4) et une barre (1) de surface extérieure lisse, caracterisé par ce que le vis à pas (3) est pourva d'un cavet cylindrique (8).

2. Système de fixation de colonne, selon la revendication première, caracterisé en ce que le tige pédiculaire (5) possède un filetage variable avec plus d' hauteur de dent à la pointe et moins d'hauteur à la fin du filetage, ceci dû à ce que le noyau est conique et que l'extérieur a un diamètre constant.

3. Système de fixation de colonne, selon les revendications première et antérieure, caracterisé en ce que le vis à pas (3) incorpore un cavet (8) qui s'adapte à la forme de la barre (1) lisse et qui est l'élément de pression avec la barre (1), lesquels excercent la force sur le dispositif quand il est bloqué.

4. Système de fixation de colonne, selon la revendication première, caracterisé en ce que le vis à pas (3) possède un moyen de guidage (7) qui permet la position unique, après l'avoir placé dans l'accouplement (2).

5. Système de fixation de colonne, selon la revendication première, caracterisé en ce que le vis à pas (3) est préformé de manière à entraîner un déplacement du vis, lorsqu'on actionne sur lui, déplacement qui exerce une force sur la barre (1), en obtenant le blocage du système, le vis à pas (3) consiste en une extrémité filetée sur lequel agit l'écrou (4), qui sert de butée sur l'accouplement (2) et traîne le vis à pas (3) en haut, en obtenant la pression désirée entre la barre (1) et le cavet (8).
